# EUROPÄISCHE PATENTANMELDUNG

(11) **EP 2 045 236 A1**
(43) Veröffentlichungstag der Anmeldung: **08.04.2009**
(21) Anmeldenummer: 07016606.1
(22) Anmeldetag: 24.08.2007
(51) Int. Cl.: C07C 317/24, A01N 41/10

(54) **Thermodynamisch stabile Kristallmodifikation von 2-({2-Chlor-4-(methylsulfonyl)-3-[(2,2,2-trifluorethoxy)methyl]phenyl}carbonyl)cyclohexan-1,3-dion**

(71) Anmelder: Bayer CropScience Aktiengesellschaft, 40789 Monheim (DE)
(72) Erfinder: Olenik, Britta, Dr., 46242 Bottrop (DE); van Almsick, Andreas, Dr., 61184 Karben (DE); Hinz, Martin-Holger, Dr., 42499 Hückeswagen (DE); Patel, Smita, Dr., 65817 Eppstein-Bremthal (DE); Sixl, Frank, Dr., 26529 Rechtsupweg (DE); Thielking, Gerhard, Dr., 51399 Burscheid (DE); Dworacek, Sylvia, 42653 Solingen (DE)

(57) **Zusammenfassung**

Es wird eine thermodynamisch stabile Kristallmodifikation des herbiziden Wirkstoffs 2-({2-Chlor-4-(methylsulfonyl)-3-[(2,2,2-trifluorethoxy)methyl]phenyl}carbonyl) cyclohexan-1,3-dion (Tembotrione) beschrieben. Diese thermodynamisch stabile Kristallmodifikation weist besondere Vorteile in der Stabilität von Suspensionformulierungen auf.

## Beschreibung

Die Erfindung betrifft das technische Gebiet der Pflanzenschutzmittel.

Spezieller betrifft sie eine thermodynamisch stabile Kristallmodifikation von 2-({2-Chlor-4-(methylsulfonyl)-3-[(2,2,2-trifluorethoxy)methyl]phenyl}carbonyl)cyclohexan-1,3-dion der Formel 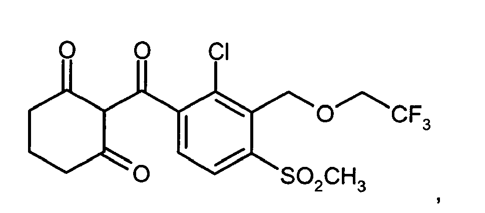 (im Folgenden als Tembotrione bezeichnet), Verfahren zu ihrer Herstellung und deren Verwendung als Herbizid.

Es ist bekannt, dass einige organische Verbindungen in nur einer Kristallstruktur, andere (sog. Polymorphe) in verschiedenen Kristallstrukturen auftreten können, siehe dazu beispielsweise J. Bernstein, R.J. Davey, J.O. Henck, Angew. Chem. Int. Ed., 1999, 38, 3440-3461. So sind aus EP 1 314 724 A1 zwei Kristallstrukturen des herbiziden Wirkstoffs Sulcotrione bekannt.

Das beispielsweise aus WO 00/21924 (dort Beispiel Nr. 3 in Tabelle 1) bekannte Tembotrione besitzt herbizide Eigenschaften und eignet sich für die Herstellung von Pflanzenschutzmitteln, die zur Unkrautbekämpfung herangezogen werden. Jedoch hat sich gezeigt, dass das gemäß der Offenbarung von WO 00/21924 herstellbare Tembotrione sich nicht für die Herstellung von anwenderfreundlichen Darreichungsformen eignet. Anwenderfreundliche Darreichungsformen sind beispielsweise Suspensionsformulierungen, in denen Tembotrione feinvermahlen in fester Form vorliegt. In der praktischen Ausprüfung hat sich gezeigt, dass das gemäß der Offenbarung von WO 00/21924 herstellbare Tembotrione in Suspensionsformulierungen zu Kristallwachstum und in Folge dessen zu Ausklumpen und Ausfällungen führt, sodass die Suspensionsformulierung unbrauchbar wird. Das Kristallwachstum kann spontan auftreten oder über einen längeren Zeitraum erfolgen und kann nicht vorhergesagt werden.

Aufgabe der vorliegenden Erfindung war die Bereitstellung einer Modifikation von Tembotrione, die diese Nachteile überwindet und für die Herstellung einer über einen längeren Zeitraum lagerstabilen Suspensionsformulierung geeignet ist.

Es wurde eine thermodynamisch stabile in einem orthorhombischen System kristallisierende Modifikation von Tembotrione gefunden, die die oben genannten Nachteile nicht aufweist und daher für die Herstellung von Suspensionsformulierungen wie Suspokonzentraten, Suspoemulsionen und Öldispersionen besonders geeignet ist.

Ein Gegenstand der Erfindung ist daher eine in einem orthorhombischen System kristallisierende Modifikation von 2-({2-Chlor-4-(methylsulfonyl)-3-[(2,2,2-trifluorethoxy)methyl]phenyl}carbonyl)cyclohexan-1,3-dion (Tembotrione).

Im Rahmen der vorliegenden Erfindung wurde festgestellt, dass Tembotrione außer der in einem orthorhombischen System kristallisierenden stabilen Modifikation auch in mindestens zwei metastabilen Modifikationen auftritt.

Im Folgenden werden die Begriffe "stabile Modifikation I", "stabile Kristallmodifikation I", "thermodynamisch stabile Modifikation I" und "thermodynamisch stabile Kristallmodifikation I" als gleichbedeutend verstanden.

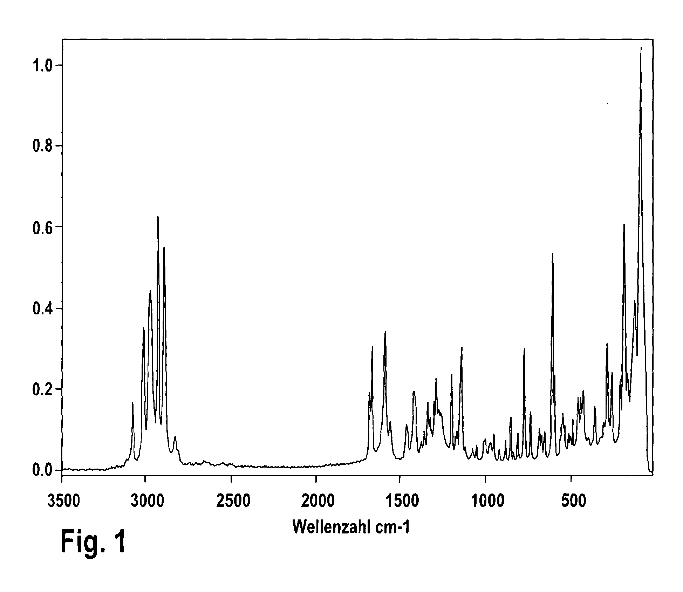

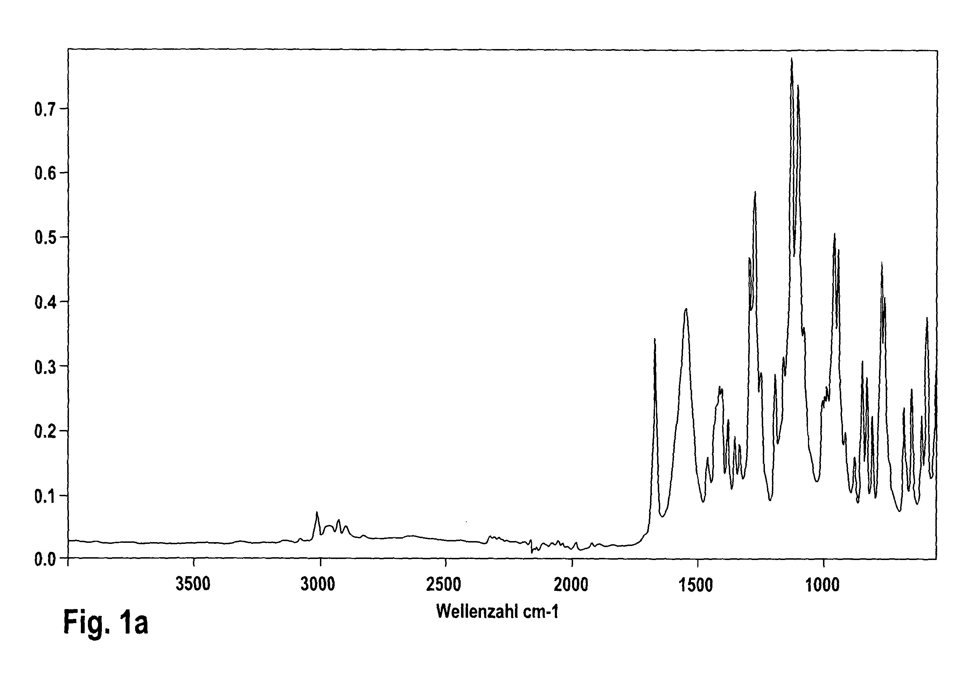

Die stabile Modifikation hat einen Schmelzpunkt von 124,0°C, ein charakteristisches Raman-Spektrum (Abb. 1) und ein charakteristisches Infrarot-Spektrum (Abb. 1a). Sie wird nachfolgend als Kristallmodifikation I bezeichnet.

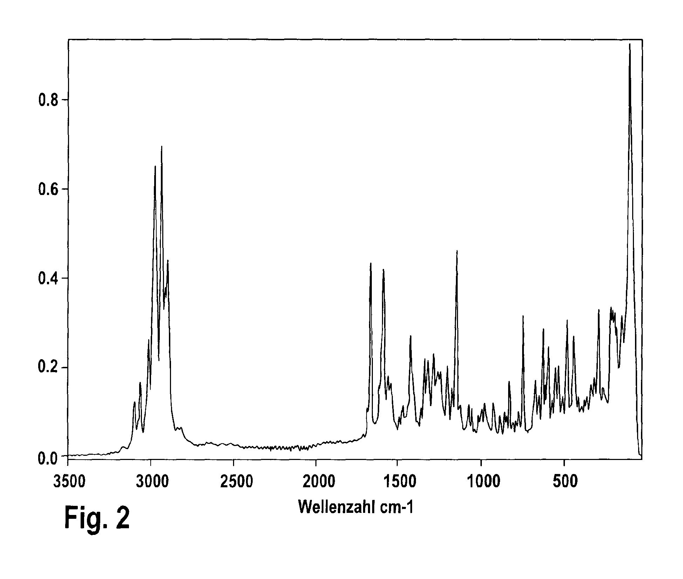

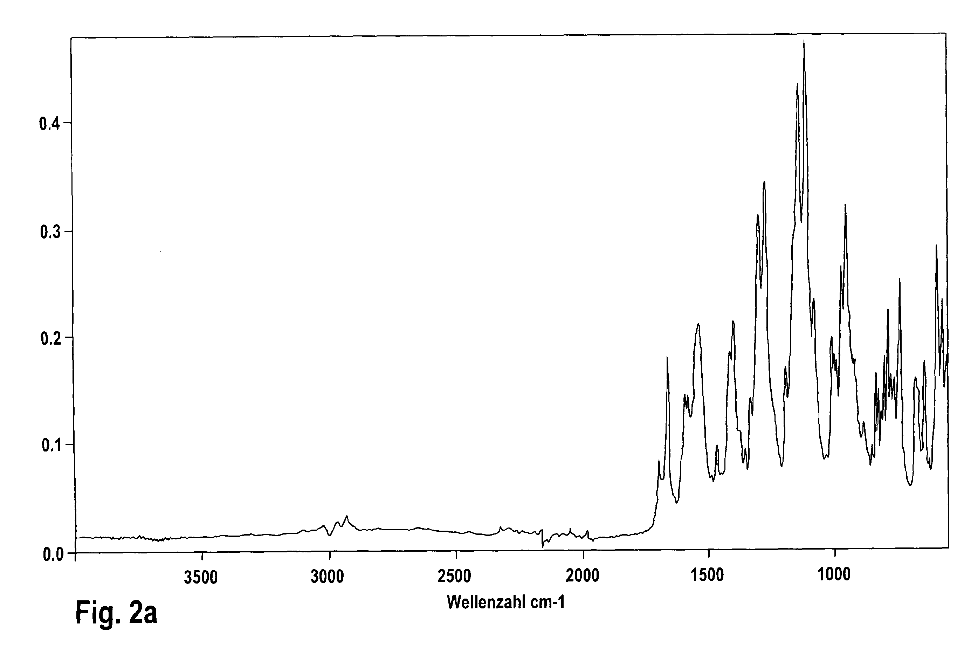

Die eine metastabile Modifikation hat einen Schmelzpunkt von 123,9°C, ein charakteristisches Raman-Spektrum (Abb. 2) und ein charakteristisches Infrarot-Spektrum (Abb. 2a). Sie wird nachfolgend als Kristallmodifikation II bezeichnet.

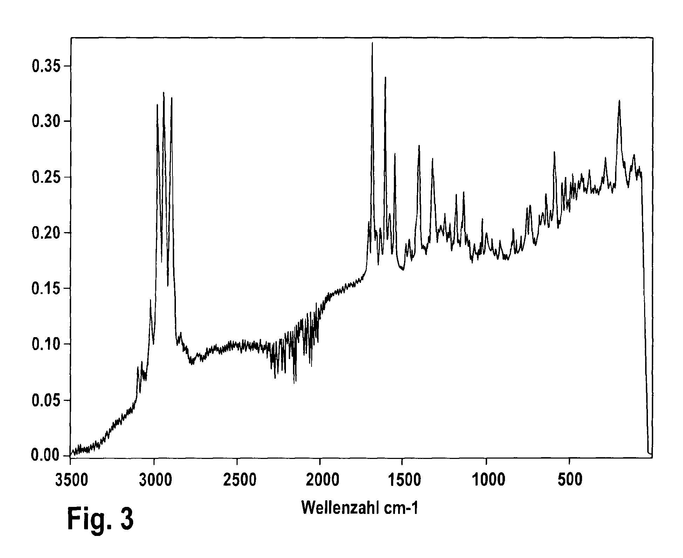

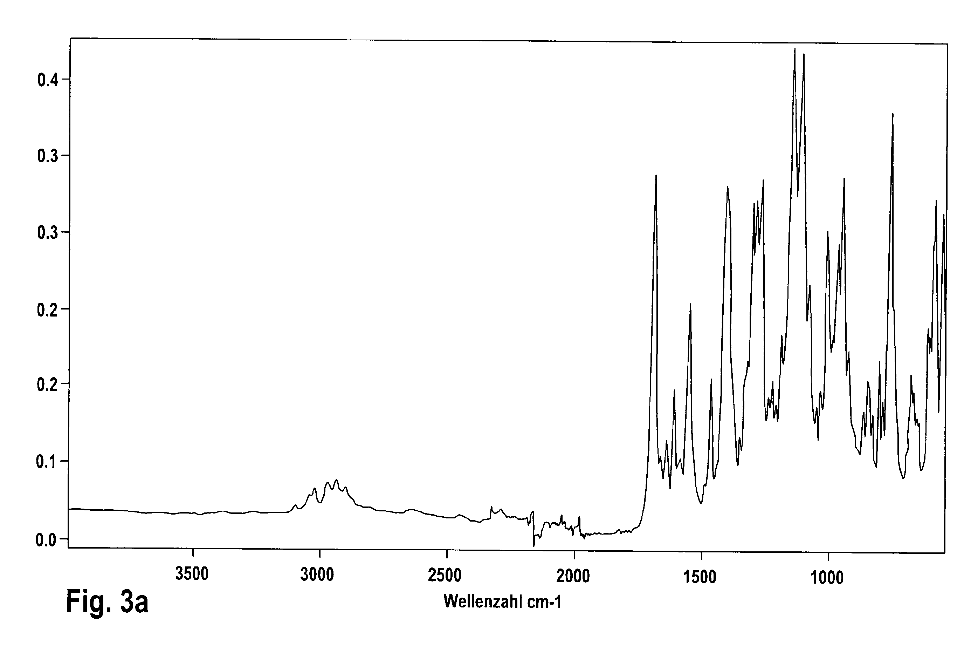

Die zweite metastabile Modifikation hat einen Schmelzpunkt von 121,6 °C, ein charakteristisches Raman-Spektrum (Abb. 3) und ein charakteristisches Infrarot-Spektrum (Abb. 3a). Sie wird nachfolgend als Kristallmodifikation III bezeichnet.

Die Einkristallstrukturanalyse der Kristallmodifikation I von Tembotrione zeigt für diese Kristallmodifikation charakteristische Bindungswinkel und Bindungslängen, die in Tabelle 3 angegeben sind.

Die Einkristallstrukturanalyse der Kristallmodifikation II von Tembotrione zeigt für diese Kristallmodifikation charakteristische Bindungswinkel und Bindungslängen, die in Tabelle 4 angegeben sind.

Die Röntgen-Pulver-Diffraktometrie der Kristallmodifikation I von Tembotrione zeigt für diese Kristallmodifikation charakteristische Peaks, die in Tabelle 5 angegeben sind.

Die Röntgen-Pulver-Diffraktometrie der Kristallmodifikation II von Tembotrione zeigt für diese Kristallmodifikation charakteristische Peaks, die in Tabelle 6 angegeben sind.

Weitere kristallographische Daten der Kristallmodifikationen I und II von Tembotrione sind in Tabelle 7 angegeben.

Beschreibung der Abbildungen Abbildung 1 zeigt das Raman-Spektrum der Kristallmodifikation I von Tembotrione. Die Werte der Bandenmaxima in Wellenzahlen sind in Tabelle 1 aufgeführt. Abbildung 1a zeigt das Infrarot-Spektrum der Kristallmodifikation I von Tembotrione. Die Werte der Bandenmaxima in Wellenzahlen sind in Tabelle 2 aufgeführt. Abbildung 2 zeigt das Raman-Spektrum der Kristallmodifikation II von Tembotrione. Die Werte der Bandenmaxima in Wellenzahlen sind in Tabelle 1 aufgeführt. Abbildung 2a zeigt das Infrarot-Spektrum der Kristallmodifikation II von Tembotrione. Die Werte der Bandenmaxima in Wellenzahlen sind in Tabelle 2 aufgeführt. Abbildung 3 zeigt das Raman-Spektrum der Kristallmodifikation III von Tembotrione. Die Werte der Bandenmaxima in Wellenzahlen sind in Tabelle 1 aufgeführt. Abbildung 3a zeigt das Infrarot-Spektrum der Kristallmodifikation III von Tembotrione. Die Werte der Bandenmaxima in Wellenzahlen sind in Tabelle 2 aufgeführt.

**Tabelle 1: Bandenmaxima der Raman-Spektren in [cm⁻¹]**

| Kristallmodifikation I | | Kristallmodifikation II | | Kristallmodifikation III | |
|---|---|---|---|---|---|
| 91 | 921 | 94 | 976 | 85 | 867 |
| 130 | 952 | 146 | 992 | 92 | 891 |
| 171 | 968 | 181 | 1015 | 95 | 899 |
| 192 | 1002 | 203 | 1055 | 108 | 927 |
| 213 | 1053 | 215 | 1072 | 124 | 957 |
| 265 | 1075 | 253 | 1123 | 145 | 977 |
| 279 | 1116 | 292 | 1144 | 215 | 1004 |
| 291 | 1141 | 313 | 1171 | 269 | 1008 |
| 311 | 1166 | 334 | 1196 | 299 | 1037 |
| 363 | 1176 | 360 | 1239 | 315 | 1050 |
| 402 | 1197 | 378 | 1255 | 361 | 1081 |
| 431 | 1272 | 393 | 1281 | 371 | 1112 |
| 445 | 1289 | 416 | 1315 | 392 | 1129 |
| 460 | 1300 | 441 | 1335 | 434 | 1144 |
| 490 | 1325 | 481 | 1356 | 440 | 1154 |
| 507 | 1337 | 508 | 1420 | 449 | 1188 |
| 517 | 1358 | 535 | 1435 | 456 | 1226 |
| 540 | 1377 | 550 | 1466 | 474 | 1238 |
| 550 | 1418 | 571 | 1487 | 480 | 1254 |
| 599 | 1461 | 594 | 1538 | 493 | 1279 |
| 613 | 1557 | 626 | 1554 | 506 | 1286 |
| 655 | 1587 | 649 | 1584 | 513 | 1326 |
| 675 | 1665 | 672 | 1610 | 522 | 1353 |
| 687 | 1679 | 746 | 1662 | 539 | 1410 |
| 738 | 2888 | 782 | 1681 | 550 | 1445 |
| 775 | 2925 | 791 | 2895 | 557 | 1453 |
| 813 | 2971 | 807 | 2908 | 575 | 1469 |
| 836 | 2978 | 830 | 2928 | 579 | 1487 |
| 853 | 3010 | 845 | 2971 | 591 | 1553 |
| 883 | 3075 | 855 | 3011 | 604 | 1583 |
| | | 885 | 3062 | 625 | 1610 |
| | | 919 | 3096 | 653 | 1637 |
| | | 927 | | 670 | 1663 |
| | | | | 684 | 1684 |
| | | | | 694 | 1711 |
| | | | | 707 | 2838 |
| | | | | 722 | 2892 |
| | | | | 750 | 2937 |
| | | | | 768 | 2973 |
| | | | | 805 | 3020 |
| | | | | 832 | 3073 |
| | | | | 850 | 3099 |

**Tabelle 2: Bandenmaxima der Infrarot-Spektren in [cm⁻¹]**

| Kristallmodifikation I | | Kristallmodifikation II | | Kristallmodifikation III | |
|---|---|---|---|---|---|
| 593 | 1164 | 555 | 1033 | 555 | 1154 |
| 612 | 1196 | 571 | 1082 | 585 | 1190 |
| 654 | 1253 | 592 | 1116 | 604 | 1211 |
| 686 | 1282 | 625 | 1142 | 611 | 1226 |
| 765 | 1298 | 645 | 1194 | 666 | 1238 |
| 777 | 1336 | 680 | 1275 | 678 | 1273 |
| 786 | 1356 | 743 | 1299 | 766 | 1293 |
| 813 | 1386 | 766 | 1333 | 778 | 1309 |
| 836 | 1409 | 777 | 1353 | 791 | 1325 |
| 853 | 1417 | 789 | 1375 | 804 | 1350 |
| 883 | 1461 | 804 | 1400 | 831 | 1414 |
| 921 | 1553 | 816 | 1413 | 851 | 1467 |
| 951 | 1675 | 828 | 1464 | 867 | 1553 |
| 966 | 2897 | 838 | 1538 | 927 | 1584 |
| 994 | 2926 | 853 | 1580 | 955 | 1611 |
| 1010 | 2959 | 883 | 1591 | 971 | 1641 |
| 1085 | 3010 | 918 | 1660 | 988 | 1662 |
| 1112 | 3075 | 927 | 1695 | 1016 | 1696 |
| 1138 | | 956 | 2929 | 1034 | 2901 |
| | | 973 | 2968 | 1052 | 2938 |
| | | 991 | 3023 | 1081 | 2972 |
| | | 1001 | 3098 | 1119 | 3023 |
| | | 1012 | | 1144 | 3099 |

**Tabelle 3: Bindungslängen [Å] und Bindungswinkel [°] der Kristallmodifikation I**

| Bindung | Å(°) | Bindung | Å(°) |
|---|---|---|---|
| Cl(1)-C(3) | 1.732(2) | O(6)-C(17) | 1.305(3) |
| S(1)-O(2) | 1.4377(17) | C(9)-C(10) | 1.486(3) |
| S(1)-O(1) | 1.439(2) | C(11)-C(12) | 1.441(3) |
| S(1)-C(7) | 1.755(3) | C(12)-C(17) | 1.395(3) |
| S(1)-C(1) | 1.784(2) | C(12)-C(13) | 1.472(3) |
| C(1)-C(6) | 1.391(3) | C(13)-C(14) | 1.516(3) |
| C(1)-C(2) | 1.405(3) | C(14)-C(15) | 1.505(4) |
| F(1)-C(10) | 1.328(3) | C(15)-C(16) | 1.515(4) |
| C(2)-C(3) | 1.400(3) | C(16)-C(17) | 1.491(3) |
| C(2)-C(8) | 1.511(3) | O(3)-C(9)-C(10) | 107.7(2) |
| F(2)-C(10) | 1.316(3) | F(2)-C(10)-F(1) | 105.9(3) |
| C(3)-C(4) | 1.390(3) | F(2)-C(10)-F(3) | 106.8(2) |
| O(3)-C(9) | 1.407(4) | F(1)-C(10)-F(3) | 106.6(2) |
| O(3)-C(8) | 1.427(3) | F(2)-C(10)-C(9) | 114.6(2) |
| F(3)-C(10) | 1.331(4) | F(1)-C(10)-C(9) | 111.3(2) |
| O(4)-C(11) | 1.245(3) | F(3)-C(10)-C(9) | 111.2(3) |
| C(4)-C(5) | 1.387(3) | O(4)-C(11)-C(12) | 121.6(2) |
| C(4)-C(11) | 1.502(3) | O(4)-C(11)-C(4) | 115.24(19) |
| O(5)-C(13) | 1.219(3) | C(12)-C(11)-C(4) | 123.02(19) |
| C(5)-C(6) | 1.387(3) | C(17)-C(12)-C(11) | 117.8(2) |
| O(2)-S(1)-O(1) | 117.41(13) | C(17)-C(12)-C(13) | 119.7(2) |
| O(2)-S(1)-C(7) | 109.36(14) | C(11)-C(12)-C(13) | 122.49(19) |
| O(1)-S(1)-C(7) | 108.37(12) | O(5)-C(13)-C(12) | 122.2(2) |
| O(2)-S(1)-C(1) | 109.85(10) | O(5)-C(13)-C(14) | 120.4(2) |
| O(1)-S(1)-C(1) | 107.25(11) | C(12)-C(13)-C(14) | 117.2(2) |
| C(7)-S(1)-C(1) | 103.72(12) | C(15)-C(14)-C(13) | 114.9(2) |
| C(6)-C(1)-C(2) | 121.89(19) | C(14)-C(15)-C(16) | 110.0(2) |
| C(6)-C(1)-S(1) | 114.88(16) | C(17)-C(16)-C(15) | 111.2(2) |
| C(2)-C(1)-S(1) | 123.15(16) | O(6)-C(17)-C(12) | 122.0(2) |
| C(3)-C(2)-C(1) | 116.25(19) | O(6)-C(17)-C(16) | 115.4(2) |
| C(3)-C(2)-C(8) | 118.4(2) | C(12)-C(17)-C(16) | 122.6(2) |
| C(1)-C(2)-C(8) | 125.3(2) | | |
| C(4)-C(3)-C(2) | 122.7(2) | | |
| C(4)-C(3)-Cl(1) | 116.81 (16) | | |
| C(2)-C(3)-Cl(1) | 120.43(17) | | |
| C(9)-O(3)-C(8) | 112.98(19) | | |
| C(5)-C(4)-C(3) | 119.1(2) | | |
| C(5)-C(4)-C(11) | 119.83(19) | | |
| C(3)-C(4)-C(11) | 120.7(2) | | |
| C(6)-C(5)-C(4) | 120.1 (2) | | |
| C(5)-C(6)-C(1) | 119.8(2) | | |
| O(3)-C(8)-C(2) | 111.96(18) | | |

**Tabelle 4: Bindungslängen [Å] und Bindungswinkel der Kristallmodifikation II**

| Bindung | Å(°) | Bindung | Å(°) |
|---|---|---|---|
| Cl(1)-C(3) | 1.7352(16) | C(9B)-C(10B) | 1.505(5) |
| S(1)-O(1) | 1.4294(14) | C(10B)-F(3B) | 1.323(5) |
| S(1)-O(2) | 1.4297(16) | C(10B)-F(1B) | 1.328(5) |
| S(1)-C(7) | 1.743(3) | C(10B)-F(2B) | 1.361(11) |
| S(1)-C(1) | 1.7843(16) | C(11)-C(12) | 1.444(2) |
| C(1)-C(6) | 1.381(2) | C(12)-C(13) | 1.390(2) |
| C(1)-C(2) | 1.407(2) | C(12)-C(17) | 1.469(2) |
| C(2)-C(3) | 1.391(2) | C(13)-C(14) | 1.480(2) |
| C(2)-C(8) | 1.506(2) | C(14)-C(15) | 1.510(3) |
| C(3)-C(4) | 1.392(2) | C(15)-C(16) | 1.506(3) |
| O(4)-C(11) | 1.243(2) | C(16)-C(17) | 1.512(2) |
| C(4)-C(5) | 1.384(2) | F(2A)-C(10A)-F(1A) | 106.2(6) |
| C(4)-C(11) | 1.505(2) | F(3A)-C(10A)-C(9A) | 114.0(9) |
| O(5)-C(13) | 1.312(2) | F(2A)-C(10A)-C(9A) | 112.2(5) |
| C(5)-C(6) | 1.380(2) | F(1A)-C(10A)-C(9A) | 113.8(5) |
| O(6)-C(17) | 1.220(2) | C(9B)-O(3B)-C(8) | 113.6(2) |
| C(8)-O(3A) | 1.381(4) | O(3B)-C(9B)-C(10B) | 112.3(3) |
| C(8)-O(3B) | 1.495(3) | F(3B)-C(10B)-F(1B) | 106.5(4) |
| O(3A)-C(9A) | 1.396(5) | F(3B)-C(10B)-F(2B) | 102.9(6) |
| C(9A)-C(10A) | 1.491(8) | F(1B)-C(10B)-F(2B) | 109.5(7) |
| C(10A)-F(3A) | 1.267(16) | F(3B)-C(10B)-C(9B) | 112.5(3) |
| C(10A)-F(2A) | 1.330(8) | F(1B)-C(10B)-C(9B) | 112.3(3) |
| C(10A)-F(1A) | 1.340(7) | F(2B)-C(10B)-C(9B) | 112.7(5) |
| O(3B)-C(9B) | 1.406(4) | O(4)-C(11)-C(12) | 122.17(15) |
| O(1)-S(1)-O(2) | 117.29(10) | O(4)-C(11)-C(4) | 116.88(14) |
| O(1)-S(1)-C(7) | 109.74(15) | C(12)-C(11)-C(4) | 120.93(14) |
| O(2)-S(1)-C(7) | 107.43(18) | C(13)-C(12)-C(11) | 118.64(14) |
| O(1)-S(1)-C(1) | 111.12(8) | C(13)-C(12)-C(17) | 119.17(14) |
| O(2)-S(1)-C(1) | 107.13(8) | C(11)-C(12)-C(17) | 122.10(14) |
| C(7)-S(1)-C(1) | 103.11(11) | O(5)-C(13)-C(12) | 122.28(15) |
| C(6)-C(1)-C(2) | 121.53(15) | O(5)-C(13)-C(14) | 114.49(15) |
| C(6)-C(1)-S(1) | 115.28(13) | C(12)-C(13)-C(14) | 123.23(15) |
| C(2)-C(1)-S(1) | 123.08(12) | C(13)-C(14)-C(15) | 111.94(15) |
| C(3)-C(2)-C(1) | 116.11 (14) | C(16)-C(15)-C(14) | 110.06(16) |
| C(3)-C(2)-C(8) | 120.47(14) | C(15)-C(16)-C(17) | 113.94(15) |
| C(1)-C(2)-C(8) | 123.42(14) | O(6)-C(17)-C(12) | 122.32(14) |
| C(2)-C(3)-C(4) | 122.82(15) | O(6)-C(17)-C(16) | 119.85(15) |
| C(2)-C(3)-Cl(1) | 120.15(12) | C(12)-C(17)-C(16) | 117.72(14) |
| C(4)-C(3)-Cl(1) | 117.03(12) | | |
| C(5)-C(4)-C(3) | 119.20(15) | | |
| C(5)-C(4)-C(11) | 119.03(14) | | |
| C(3)-C(4)-C(11) | 121.76(14) | | |
| C(6)-C(5)-C(4) | 119.46(15) | | |
| C(5)-C(6)-C(1) | 120.70(16) | | |
| O(3A)-C(8)-O(3B) | 30.21(15) | | |
| O(3A)-C(8)-C(2) | 110.68(18) | | |
| O(3B)-C(8)-C(2) | 104.49(15) | | |
| C(8)-O(3A)-C(9A) | 116.4(3) | | |
| O(3A)-C(9A)-C(10A) | 112.2(4) | | |
| F(3A)-C(10A)-F(2A) | 110.4(11) | | |
| F(3A)-C(10A)-F(1A) | 99.2(10) | | |

**Tabelle 5: Röntgen-Pulver-Diffraktometrie Muster der Kristallmodifikation I von Tembotrione [2θ]**

| | | | |
|---|---|---|---|
| 7.3765 | 20.8117 | 26.6207 | 32.4069 |
| 8.0674 | 21.1093 | 27.2879 | 32.8121 |
| 10.7988 | 21.5838 | 27.4979 | 33.1960 |
| 13.5030 | 21.6983 | 27.9884 | 33.5965 |
| 14.7553 | 23.5072 | 28.2728 | 34.4007 |
| 16.4462 | 23.9969 | 28.5989 | 35.1147 |
| 16.6192 | 24.3808 | 29.0017 | 35.7136 |
| 17.0512 | 24.8173 | 30.0318 | 35.9960 |
| 17.1467 | 25.0316 | 30.2456 | 36.4372 |
| 17.6444 | 25.2513 | 30.6058 | 36.7974 |
| 17.7792 | 25.5214 | 30.7628 | |
| 19.3008 | 25.7119 | 31.2971 | |
| 20.4034 | 25.9248 | 31.6675 | |

**Tabelle 6: Röntgen-Pulver-Diffraktometrie Muster der Kristallmodifikation II von Tembotrione [2θ]**

| | | | |
|---|---|---|---|
| 7.3765 | 20.8117 | 26.6207 | 32.4069 |
| 8.0674 | 21.1093 | 27.2879 | 32.8121 |
| 10.7988 | 21.5838 | 27.4979 | 33.1960 |
| 13.5030 | 21.6983 | 27.9884 | 33.5965 |
| 14.7553 | 23.5072 | 28.2728 | 34.4007 |
| 16.4462 | 23.9969 | 28.5989 | 35.1147 |
| 16.6192 | 24.3808 | 29.0017 | 35.7136 |
| 17.0512 | 24.8173 | 30.0318 | 35.9960 |
| 17.1467 | 25.0316 | 30.2456 | 36.4372 |
| 17.6444 | 25.2513 | 30.6058 | 36.7974 |
| 17.7792 | 25.5214 | 30.7628 | |
| 19.3008 | 25.7119 | 31.2971 | |
| 20.4034 | 25.9248 | 31.6675 | |

**Tabelle 7: Kristallographische Daten**

| | Kristallmodifikation I | | Kristallmodifikation II | |
|---|---|---|---|---|
| Symmetrietyp | orthorhombisch | | monoklin | |
| Raumgruppe | *P*na2₁ | | P2(1)/n | |
| Dimensionen der | a = 31,1647(18) Å | α = 90° | a = 15,8491 (5) (18) Å | α = 90° |
| Elementarzelle | b = 10,3522(6) Å | β = 90° | b =7,1164(2) Å | β = 95,721° |
| | c = 5,5449(3) Å | γ = 90° | c = 16,1656(6) Å | γ = 90° |
| Volumen der Elementarzelle | 1788,91(18) Å3 | | 1814,21(10) Å3 | |
| Koordinationszahl | 4 | | 4 | |
| Dichte (rechnerisch) | 1,637 Mg/m³ | | 1,614 Mg/m³ | |

### Beschreibung der Erfindung

Die Schmelzpunkte wurden mittels DSC (Pyris 1 der Fa. Perkin Elmer, Heizrate 10 K min⁻¹ermittelt. Zur Bestimmung der Raman-Spektren wurden mittels eines RFS 100/S FT-Raman der Fa. Bruker von jeder Partie mindestens zwei Spektren mit jeweils 128 Scans aufgenommen. Die Infrarot-Spektren wurden mit einem FT-IR-Spektrometer (Fa. Bruker Tensor 37) mit jeweils 64 Scans aufgenommen.
Die Feststoffdichte wurde nach der Dichtebestimmungsmethode SOP 5024 mit dem Ultrapyknometer 1000 T der Fa. Quanta-Chrome bestimmt bzw. aus der Einkristallröntgenstrukturanalyse (EKS) ermittelt. Die Einkristallröntgenstrukturanalyse wurde durch Verwendung einer Drehanode M18X-HF mit MoKα-Strahlung von MACScience Co und einen SMART-CCD-1000-Detektor von Bruker-AXS bestimmt. Die Daten wurden mit den Programmen SAINT-NT V 5.0 (Datenreduktion, Bruker-AXS) und SADABS (Absorptionskorrektur, Bruker-AXS) bearbeitet. Die Strukturlösung und Verfeinerung wurde mit SHELXTL NT-Version V5.1 durchgeführt.

### Herstellung

Tembotrione an sich kann beispielsweise nach einem der in WO 00/21924 genannten Verfahren hergestellt werden. In Abhängigkeit von der Art des im letzten Reinigungsschritt verwendeten Lösungsmittels und der Temperaturführung kristallisiert Tembotrione üblicherweise als Gemisch oder in Form einer reinen der hier beschriebenen metastabilen Kristallmodifikationen II und III aus.

Die thermodynamisch stabile Kristallmodifikation I von Tembotrione kann in allgemeiner Weise so hergestellt werden, dass man die nach WO 00/21924 erhältliche Kristallmodifikation II und III von Tembotrione beziehungsweise Gemische davon in einem geeigneten Lösungsmitteln suspendiert und/oder löst und bis zur quantitativen Umwandlung in die thermodynamisch stabile Kristallmodifikation I bei Temperaturen von 0°C bis 80°C behandelt.

Ein weiterer Gegenstand der Erfindung ist somit ein Verfahren zur Herstellung der thermodynamisch stabilen Kristallmodifikation I von Tembotrione, wobei man die Kristallmodifikationen II und III von Tembotrione beziehungsweise Gemische davon in Lösungsmitteln suspendiert und/oder löst und bis zur quantitativen Umwandlung in die thermodynamisch stabile Kristallmodifikation I bei Temperaturen von 0°C bis 80°C behandelt.

Geeignete Lösungsmittel, die in diesem Verfahren verwendet werden können, sind z.B. niedere Alkohole wie Methanol, Ethanol, 2-Propanol oder Ketone wie Aceton, 2-Butanon, die auch in Mischung mit Wasser verwendet werden können. Als niedere Alkohole bzw. Ketone werden hier solche Verbindungen bezeichnet, die eins bis zehn Kohlenstoffatome aufweisen, bevorzugt eins bis fünf Kohlenstoffatome. Weitere geeignete Lösungsmittel sind Benzol, Toluol und Chlorbenzol.

Die Umwandlung in die thermodynamisch stabile Kristallmodifikation I erfolgt bei Temperaturen kleiner 100°C, bevorzugt bei Temperaturen von 0°C bis 80°C, besonders bevorzugt bei Temperaturen von 60°C bis 80°C, ganz besonders bevorzugt bei Temperaturen von 50°C bis 80°C. Die Dauer der Umwandlung hängt ab von der Temperatur und der Art des Lösungsmittels. Weiterhin hängt die Dauer der Umwandlung davon ab, ob Impfkristalle der Kristallmodifikation I verwendet werden. Im Allgemeinen kann die Umwandlung zur Kristallmodifikation I bei vollständiger Auflösung der Kristalle der Kristallmodifikationen II und III beziehungsweise Gemischen davon bei erhöhter Temperatur durch Kühlungskristallisation zur Raumtemperatur ohne die Verwendung von Impfkristallen direkt erzielt werden. Das Abkühlen auf Raumtemperatur erfolgt vorzugsweise mit einer Kühlrate von kleiner 25°C, besonders bevorzugt mit einer Kühlrate von kleiner 20°C. Die Umwandlung einer Suspension von Kristallmodifikationen II und III beziehungsweise Gemischen davon kann in der Regel ohne die Verwendung von Impfkristallen in einem Zeitraum von 14 Tagen herbeigeführt werden. Werden bei der Umwandlung einer Suspension Impfkristalle der Kristallmodifikation I verwendet, ist im Allgemeinen eine Behandlungsdauer von 24 bis 48 Stunden ausreichend, um eine quantitative Umwandlung der Kristalle in die Kristallmodifikation I zu erreichen.

Die erhaltenen Kristalle der Kristallmodifikation I werden schließlich abgetrennt und zur Entfernung des Lösungsmittels bei Raumtemperatur oder erhöhter Temperatur bis zur Gewichtskonstanz getrocknet.

Die stabile Kristallmodifikation I kann auch durch Mahlen unter hohem Druck aus den Kristallmodifikationen II und III beziehungsweise Gemischen davon erhalten werden. Geeigneter Druck ist ein Druck von mindestens 5 bar.

Die Kristallmodifikation I eignet sich aufgrund ihrer Stabilität hervorragend für die Zubereitung von Formulierungen, insbesondere Suspensionsformulierungen von Pflanzenschutzmitteln. Gegenstand der Erfindung sind daher auch Pflanzenschutzmittel, welche die Kristallmodifikation I von Tembotrione alleine oder in Mischung mit Hilfs- und Trägerstoffen, sowie in Mischung mit anderen Wirkstoffen enthalten. Die Erfindung schließt auch Mischungen der Kristallmodifikation I von Tembotrione mit den Kristallmodifikationen II und III von Tembotrione ein, z.B. solche, die an irgendeiner Stelle des erfindungsgemäßen Umwandlungsverfahrens der Kristallmodifikationen II und III beziehungsweise Gemischen davon in die Kristallmodifikation I auftreten. Bevorzugt wird eine Wirkstoffqualität mit mehr als 20 Gew.-% der Kristallmodifikation I von Tembotrione, besonders bevorzugt mit mehr als 90 Gew.-%, ganz besonders bevorzugt mit mehr als 95 Gew.-% und am meisten bevorzugt mit mehr als als 98 Gew.-%.

Gegebenenfalls wird Tembotrione in der Kristallmodifikation I mit einem oder mehreren anderen Herbiziden gemischt. Auch solche Mischungen profitieren von den vorteilhaften Eigenschaften der erfindungsgemäßen Kristallmodifikation I.

Aufgrund ihrer Stabilität eignet sich die Kristallmodifikation I von Tembotrione ganz allgemein als Ausgangsmaterial für die Herstellung jedweder Tembotrione enthaltender Pflanzenschutzformulierungen, auch wenn das Tembotrione nach der Formulierung nicht mehr in dieser Form, sondern etwa in gelöster Form vorliegt.

Gegenstand der Erfindung sind daher auch Verfahren zur Herstellung von Tembotrione enthaltenden Pflanzenschutzformulierungen, welche die Kristallmodifikation I von Tembotrione verwenden sowie Tembotrione enthaltende Pflanzenschutzformulierungen, die aus der Kristallmodifikation I von Tembotrione erhalten wurden. Durch den Einsatz der Kristallmodifikation I wird die Sicherheit für Zubereitungen von Tembotrione erhöht und somit das Risiko falscher Dosierungen verringert. Die Kristallmodifikation I von Tembotrione kann in bekannter Weise in die üblichen Formulierungen überführt werden, wie Suspensionskonzentrate, kolloidale Konzentrate, dispergierbare Konzentrate, emulgierbare Konzentrate (Emulsionskonzentrate), Emulsionsbeizen, Suspensionsbeizen, Granulate, Mikrogranulate, Suspoemulsionen, Öldispersionen, wasserlösliche Granulate, wasserlösliche Konzentrate und wasserdispergierbare Granulate, unter Verwendung geeigneter Hilfs- und Trägerstoffe oder Lösemittel. Hierbei soll die wirksame Verbindung in einer Konzentration von etwa 0,5 bis 90 Gew.-% der Gesamtmischung vorhanden sein, d.h. in Mengen, die ausreichend sind, um den notwendigen Dosierungsspiegel zu erreichen. Die Formulierungen werden beispielsweise hergestellt durch Verstrecken der Kristallmodifikation I von Tembotrione mit Lösemitteln und/oder Trägerstoffen, gegebenenfalls unter Verwendung von Emulgier- und/oder Dispergiermitteln, und/oder anderen Hilfsstoffen, wie z.B. Penetrationshilfsmitteln.

Die Anwendung erfolgt in der üblichen Weise, indem die unerwünschten Pflanzen und/oder ihr Lebensraum mit dem Wirkstoff bzw. dessen Formulierung in Kontakt gebracht werden.

Tembotrione in der Kristallmodifikation I zeigt eine hervorragende herbizide Wirkung gegenüber Vertretern der Gruppe sowohl der monokotylen als auch der dikotylen Pflanzen. Gegenstand der Erfindung ist daher auch die Verwendung der Kristallmodifikation I von Tembotrione zur Herstellung eines Pflanzenschutzmittels zur Behandlung des Unkrautbefalls.

Dikotyle Pflanzen der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Monokotyle Pflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Die erfindungsgemäße Kristallmodifikation I von Tembotrione ist aufgrund ihrer hohen Verträglichkeit gegenüber Kulturpflanzen zur Bekämpfung unerwünschter Pflanzen in Kulturen von beispielsweise Weizen, Gerste, Hafer, Roggen, Reis, Mais, Zuckerrübe, Zuckerrohr, Baumwolle und Soja, insbesondere in Reis, Mais und Zuckerrohr, geeignet. Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit der erfindungsgemäßen Kristallmodifikation I von Tembotrione erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen oder Aufstreichen.

Die erfindungsgemäße Kristallmodifikation I von Tembotrione kann, wie bereits oben ausgeführt, in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent des Wirkstoffs in der erfindungsgemäßen Kristallmodifikationen I, vorzugsweise zwischen 0,5 und 90 %.

Die erfindungsgemäße Kristallmodifikation I von Tembotrione kann als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, lodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesosulfuron (-methyl, - sodium), Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise AD-67, BAS-145138, Benoxacor, Cloquintocet (-mexyl), Cyometrinil, Cyprosulfamide, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die erfindungsgemäße Kristallmodifikation I von Tembotrione kann als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäße Kristallmodifikation I von Tembotrione kann sowohl vor als auch nach dem Auflaufen der Pflanzen appliziert werden. Sie kann auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 1 kg Wirkstoff pro Hektar Bodenfläche, vorzugsweise zwischen 5 g und 500 g pro ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die sowohl durch konventionelle Züchtung, durch Mutagenese oder durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel - auch in Kombination mit anderen agrochemischen Wirkstoffen - , besseres Pflanzenwachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Soja, Kartoffel, Baumwolle, Raps sowie insbesondere Mais sowie Obstpflanzen (mit den Früchten Äpfel, Birnen, Zitrusfrüchten und Weintrauben) erwähnt, wobei insbesondere Mais, aber auch Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus *Bacillus thuringiensis* (z.B. durch die Gene CrylA(a), CrylA(b), CrylA(c), CryIIA, CrylIIIA, CrylIIIB2, Cry9c, Cry2Ab, Cry3Bb und CrylF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien v.a. Maissorten, jedoch ebenso Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien v.a. Maissorten, jedoch ebenso Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Ausführungsbeispiele

### Herstellung der thermodynamisch stabilen Kristallmodifikation I

2 g Tembotrione wurden in der Siedehitze (56°C) vollständig in Aceton gelöst und dann langsam, d.h. mit einer Kühlrate kleiner 20°C/h bis auf Raumtemperatur abgekühlt. Das Kristallisat wird abfiltriert und bei Temperaturen < 60°C getrocknet.

### Herstellung der metastabilen Kristallmodifikation II

2 g Tembotrione wurden in der Siedehitze (78°C) vollständig in Ethanol gelöst. Die klare Lösung wurde dann langsam, d.h. mit einer Kühlrate kleiner 20°C/h bis auf 7°C abgekühlt. Das Kristallisat wird abfiltriert und bei Temperaturen < 60°C getrocknet.

### Herstellung der metastabilen Kristallmodifikation III

2 g Tembotrione wurden in der Siedehitze (111°C) vollständig in Toluol gelöst. Die klare Lösung wurde dann langsam, d.h. mit einer Kühlrate kleiner 20°C/h bis auf 7°C abgekühlt. Das Kristallisat wird abfiltriert und bei Temperaturen < 60°C getrocknet.

### Stabilitätsversuche

Eine Öldispersion von Tembotrione der Kristallmodifikation I zeigt im Vergleich zu einer Öldispersion von Tembotrione der Kristallmodifikation II, III oder einem Gemisch davon, auch nach mehreren Wochen Lagerung keine Anzeichen von Ausklumpen und Ausfällungen.

## Patentansprüche

1. Kristallmodifikation von 2-({2-Chlor-4-(methylsulfonyl)-3-[(2,2,2-trifluorethoxy)methyl]phenyl}carbonyl)cyclohexan-1,3-dion (Tembotrione) in einem orthorhombischen System.

2. Kristallmodifikation nach Anspruch 1, wobei das orthorhombische System die Raumgruppe *P*na2₁ aufweist.

3. Kristallmodifikation nach Anspruch 1, worin die Kristallmodifikation ein Infrarot-Spektrum aufweist mit Bandenmaxima angegeben in cm⁻¹:
| | | | |
|---|---|---|---|
| 593 | 883 | 1196 | 1553 |
| 612 | 921 | 1253 | 1675 |
| 654 | 951 | 1282 | 2897 |
| 686 | 966 | 1298 | 2926 |
| 765 | 994 | 1336 | 2959 |
| 777 | 1010 | 1356 | 3010 |
| 786 | 1085 | 1386 | 3075 |
| 813 | 1112 | 1409 | |
| 836 | 1138 | 1417 | |
| 853 | 1164 | 1461 | |

4. Kristallmodifikation nach Anspruch 1, worin die Kristallmodifikation ein Raman-Spektrum aufweist mit Bandenmaxima angegeben in cm⁻¹:
| | | | |
|---|---|---|---|
| 91 | 507 | 921 | 1337 |
| 130 | 517 | 952 | 1358 |
| 171 | 540 | 968 | 1377 |
| 192 | 550 | 1002 | 1418 |
| 213 | 599 | 1053 | 1461 |
| 265 | 613 | 1075 | 1557 |
| 279 | 655 | 1116 | 1587 |
| 291 | 675 | 1141 | 1665 |
| 311 | 687 | 1166 | 1679 |
| 363 | 738 | 1176 | 2888 |
| 402 | 775 | 1197 | 2925 |
| 431 | 813 | 1272 | 2971 |
| 445 | 836 | 1289 | 2978 |
| 460 | 853 | 1300 | 3010 |
| 490 | 883 | 1325 | 3075 |

5. Kristallmodifikation nach Anspruch 1 mit einem Röntgen-Pulver-Diffraktometrie Muster, das folgende Peaks aufweist angegeben in Grad 2θ:
| | | | |
|---|---|---|---|
| 7.3765 | 20.8117 | 26.6207 | 32.4069 |
| 8.0674 | 21.1093 | 27.2879 | 32.8121 |
| 10.7988 | 21.5838 | 27.4979 | 33.1960 |
| 13.5030 | 21.6983 | 27.9884 | 33.5965 |
| 14.7553 | 23.5072 | 28.2728 | 34.4007 |
| 16.4462 | 23.9969 | 28.5989 | 35.1147 |
| 16.6192 | 24.3808 | 29.0017 | 35.7136 |
| 17.0512 | 24.8173 | 30.0318 | 35.9960 |
| 17.1467 | 25.0316 | 30.2456 | 36.4372 |
| 17.6444 | 25.2513 | 30.6058 | 36.7974 |
| 17.7792 | 25.5214 | 30.7628 | |
| 19.3008 | 25.7119 | 31.2971 | |
| 20.4034 | 25.9248 | 31.6675 | |

6. Kristallmodifikation nach Anspruch 1, **dadurch gekennzeichnet, dass** die Elementarzelle folgende Dimensionen aufweist:
| | |
|---|---|
| a = 31,1647(18) Å | α =90° |
| b = 10,3522(6) Å | β = 90° |
| c = 5,5449(3) Å | γ = 90°: |

7. Kristallmodifikation nach Anspruch 1 mit einem Schmelzpunkt von 124,0°C.

8. Verfahren zur Herstellung der thermodynamisch stabilen Kristallmodifikation gemäß Anspruch 1, **dadurch gekennzeichnet, dass** man
a) eine metastabile Kristallmodifikation oder ein Gemisch der metastabilen Kristallmodifikationen von Tembotrione in Lösungsmitteln suspendiert und/oder löst und
b) bis zur quantitativen Umwandlung in die thermodynamisch stabile Kristallmodifikation bei Temperaturen von 0°C bis 80°C behandelt.

9. Verfahren gemäß Anspruch 8, **dadurch gekennzeichnet, dass** als Lösungsmittel Alkohole oder Ketone verwendet werden.

10. Verfahren zur Herstellung der thermodynamisch stabilen Kristallmodifikation gemäß Anspruch 1, **dadurch gekennzeichnet, dass** man eine metastabile Kristallmodifikation oder ein Gemisch der metastabilen Kristallmodifikationen von Tembotrione unter hohem Druck, mindestens 5 bar, mahlt.

11. Herbizides Mittel, **gekennzeichnet durch** einen Gehalt an thermodynamisch stabiler Kristallmodifikation von Tembotrione gemäß einem der Ansprüche 1 bis 7 und gängigen Streckmitteln und/oder oberflächenaktiven Hilfsstoffen.

12. Herbizides Mittel, umfassend die thermodynamisch stabile Kristallmodifikation von Tembotrione gemäß einem der Ansprüche 1 bis 7 und eine metastabile Kristallmodifikation oder ein Gemisch der metastabilen Kristallmodifikationen von Tembotrione, **dadurch gekennzeichnet, dass** das Mittel mehr als 80 Gew.-% der stabilen Kristallmodifikation enthält.

13. Verwendung der thermodynamisch stabilen Kristallmodifikation von Tembotrione gemäß einem der Ansprüche 1 bis 7 oder eines Mittels gemäß Anspruch 11 oder 12 zur Bekämpfung von unerwünschten Pflanzen.

14. Verfahren zur Bekämpfung von unerwünschten Pflanzen, **dadurch gekennzeichnet, dass** man die thermodynamisch stabile Kristallmodifikation von Tembotrione gemäß einem der Ansprüche 1 bis 7 oder ein Mittel gemäß Anspruch 11 oder 12 auf die unerwünschten Pflanzen und/oder ihren Lebensraum einwirken lässt.

15. Verfahren nach Anspruch 14 zur Bekämpfung von Schadpflanzen in monokotylen Pflanzenkulturen.

16. Verfahren nach Anspruch 14 oder 15, worin die Pflanzenkulturen gentechnisch verändert oder durch Mutationsselektion erhalten sind.
